# EUROPEAN PATENT SPECIFICATION

(11) **EP 0 423 874 B1**
(45) Date of publication and mention of the grant of the patent: **09.08.1995**
(21) Application number: 90202700.2
(22) Date of filing: 11.10.1990
(51) Int. Cl.: C07C 47/21, C07C 45/50, C11B 9/00, A61K 7/46

(54) **Process for the preparation of aldehydes**
Verfahren zur Herstellung von Aldehyden
Procédé pour la préparation d'aldéhydes

(30) Priority: 18.10.1989 GB 8923433
(43) Date of publication of application: 24.04.1991
(73) Proprietor: SHELL INTERNATIONALE RESEARCH MAATSCHAPPIJ B.V., 2596 HR Den Haag (NL)
(72) Inventor: Bakker, Anke Gezina, NL-1031 CM Amsterdam (NL); Bachasingh, Anand Kumar, NL-1031 CM Amsterdam (NL)

(56) References cited:
- DE-A- 2 719 735
- DE-A- 2 724 484
- DE-A- 3 640 591
- TETRAHEDRON LETTERS. vol. 28, no. 9, 1987, OXFORD GB pages 973 - 976; L. COPPI ET AL.: 'Synthesis of 4-Halotetrahydropyrans from Allylsilanes and Carbonyl Compounds in the Presence of Lewis Acids.'
- TETRAHEDRON LETTERS. vol. 44, 1975, OXFORD GB pages3833 - 3836; J.F.NORMANT ET AL: 'Synthèse d Aldéhydes par action dOrganomagnésiens sur des Acétals Alpha-Ethyléniques en Présence de Cu (I).'
- BIOMASS vol. 12, 1987, pages 271 - 280;M.E.BORREDON ET AL.: 'Biomass as Source of Chemicals VII. - Synthesis of New Oxiranes.'

## Description

The invention relates to a process for the preparation of an aldehyde of the general formula I, 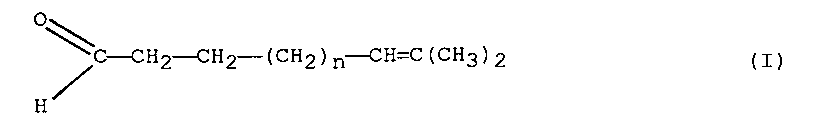
wherein n is an integer of from 2 to 8. These aldehydes can be used in fragrant compositions.

There has always been a keen interest in the preparation of compounds having fragrant properties and the use of these compounds in fragrant compositions.

Compounds of the general formula I can be made in principle by the hydroformylation of the corresponding alpha-olefins. Such a process generally results in the formation of a mixture of two isomeric aldehydes, i.e. a mixture of a n-aldehyde, being the compound wherein the formyl group can be considered to be attached to the alpha-carbon atom of its olefinic precursor, and an iso-aldehyde, being the compound wherein the formyl group can be considered to be attached to the beta-carbon atom of its olefinic precursor. The n- and the iso-aldehyde usually can be isolated from the mixture by fractional distillation.

In German patent application DE-A-2,724,484 the hydroformylation of alpha-olefins has been described. The selectivity of the reaction, however, is very low. See Example 1(b), page 7, line 7, where it is indicated that the monoaldehydes A and B are formed in the ratio 6:4. This is close to the ratio 5:5, corresponding to no selectivity at all. The catalyst used in this document is a heterogeneous catalyst. No phosphor (III) compound forms part of the catalytic system, except as the ligand in the rhodium-compound.

It has now been found that using certain homogeneous catalytic systems, which systems comprise a relatively large amount of phosphor (III) compound (in addition to the amount used as ligand in the rhodium-compound), mainly one type of aldehyde is formed, which means that a very good selectivity is obtained. Further, the conversion of the starting olefin is very high.

The present invention, therefore, relates to a process for the preparation of an aldehyde of the general formula I, 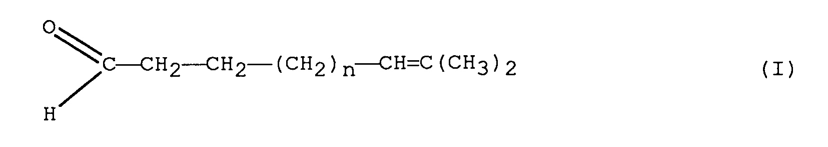
wherein n is an integer of from 2 to 8, which comprises reacting a compound of the general formula II,

CH₂=CH―(CH₂)ₙ―CH=C(CH₃)₂ (II)

wherein n is as defined herein before, with carbon monoxide and hydrogen in the presence of a homogeneous catalyst system comprising a soluble rhodium compound and a phosphor (III) compound in the range of from 50 to 10000 moles per gram atom of rhodium, and in which the temperature is in the range of from 40 to 110°C and the pressure is in the range of from 1.0 to 20 bar.

The hydroformylation catalyst is a homogeneous catalyst system comprising a soluble rhodium compound and a phosphor (III) compound, preferably a phosphine, a phosphite or phosphor (III) compounds in which the phosphor is attached to aryl and/or (cyclo)alkyl groups as well as to aryloxy and/or (cycloalkoxy groups.

The soluble rhodium compound is preferably a compound of rhodium with one or more of a hydrogen atom, carbon monoxide, a phosphor (III) compound, an olefin such as ethene or cyclooctadiene-rhodium (I) acteylacetonate, dirhodium octacarbonyl, rhodium nitrate, rhodium chloride, tetra-rhodium dodecacarbonyl or hexa rhodium hexadecacarbonyl. Other suitable rhodium compounds are carbonyl-hyrodrido-tris(triphenyl-phosphino)rhodium(I), carbonyl-chloro-bis(triphenylphosphino)-rhodium(I), acetylacetonato-carbonyl-(triphenylphosphino)rhodium(I) and acetylacetonato-bis(ethylene)rhodium(I), also known under the trade marks Rh-42, Rh-43 and Rh-91.

The phosphor (III) ligand may be a monodentate or bidentate ligand. Preferably a phosphine is used having the general formula.

P(R³)₂R⁴ (III)

in which each R³ independently represents a phenyl or naphthyl group optionally substituted by substituents selected from an alkyl, alkoxy, carboxy, trihalomethyl, cyano, dialkylamino, alkanoyl, alkanoyloxy and an alkylsulphonyl group, and a halogen atom; and R⁴ represents a group R³ or an alkyl or cycloalkyl group; any alkyl group having up to 20 carbon atoms, preferably up to 12 atoms, and any cycoalkyl group having up to 12 carbon atoms, preferably up to 6 carbon atoms. Preferably R³ represents a phenyl or naphthyl group optionally substituted by substituents selected from a methyl group, a methoxy group and a chlorine atom, and R⁴ is the same as R³ or is a phenyl group substituted by a carboxy group or is an alkyl group having from 3 to 7 carbon atoms. More preferably R³ and R⁴ both represent phenyl groups. Most preferably the phosphine ligand is triphenylphosphine.

Other suitable phosphor (III) compounds are phosphites. Very suitable use may be made of the phosphites mentioned in European patent application 306094.

The phosphor (III) compound is preferably used in a range of from 500 to 2000 moles per gram atom of rhodium.

In the process according to the invention the molar ratio of carbon monoxide to hydrogen is suitably in the range of from 2:1 to 1:2, preferably from 1.1:1 to 1:1.1. Most conveniently the ratio is 1:1 since there will then be no need to bleed off excess carbon monoxide or hydrogen from the vessel in which the reaction takes place.

The temperature at which the process is effected is preferably in the range of from 65 to 80 °C.

The pressure under which the process is effected is preferably in the range of from 1 to 3.5 bar.

The process is conveniently effected in the presence of a solvent. A suitable solvent may be selected from amides, e.g. dimethylformamide or dimethylacetamide; nitriles, e.g. acetonitrile; ethers, e.g. dioxane or tetrahydrofuran; esters, e.g. ethyl acetate; and aromatic hydrocarbons such as toluene and xylenes. Mixtures of two or more solvents may also be used.

The preparation of the branched diolefins of formula II, which may advantageously be used the in preparation of the unsaturated olefins of the present invention, is known from e.g. J. Am. Chem. Soc., 76 (1954) 4417 and J. Am. Chem. Soc., 80 (1958) 5455. The compounds of general formula (I) such as 8-methyl-7-nonenal have a fragrant odour and are thus useful as fragrances, either alone or in a fragrant compositions. Such a composition comprises a compound of general formula I and a carrier. The carrier may be, for example, talc; soap; detergent; shampoo; aerosol solution; candle; lipstick; toilet water; shaving lotion or antiperspirant. The fragrant may also be used in foodstuffs.

The fragrant composition may be optionally comprise one or more other fragrances and/or auxiliary compounds.

The amount of a fragrance to be used in a fragrant composition may vary over wide ranges, and will primarily be related to the nature of the fragrance, the nature of the composition in which the fragrance will be used as well as to the desired fragrant effect. Frequently, only as little as 0.01%m of a fragrance on fragrant composition, may already be sufficient to have a definite effect on the smell of such a composition. Usually the amount will be between 0.01 ppm and 0.1%, preferably between 0.1 ppm and 0.05%.

The invention will be further illustrated with the following example.

### Example I

### Preparation of 8-methyl-7-nonenal and 2,7-dimethyl-6 octenal

A 5 1 stainless steel autoclave equipped with a mechanical stirrer, a gas inlet tube, a thermocople and a manometer was charged with 1530 g 7-methyl-1, 6-octadiene (MOCD), 190 g. triphenylphosphine, 117 g toluene and 0.44 g Rh hydridocarbonyl tris(triphenylphosphine), which amounts of reactants correspond with Rh/ligand/diene molar ratio of 1/1500/25720.

The autoclave was flushed with an equimolar mixture of hydrogen and carbon monoxide and subsequently pressurized to 2 bar. The reactor contents were heated to 75 °C whilst maintaining the pressure at 2 bar. After 25 hours the reactor contents were allowed to cool to room temperature with the aid of gas liquid chromatography the MOCD conversion was found to be 97% mol, with a selectivity to 8-methyl-7-nonenal of 87% and to 2,7-dimethyl-6-octenal of 6%.

The heavy ends of the reaction product were removed with the aid of a rotavapor at 100 °C and 10 mbar. Subsequently the light ends were removed in an Oldershaw column having 25 plates (actual), employing a bottom temperature of 110 °C, a top temperature of 93 °C, a pressure of 30 mbar and RID=7.5 (reflux/distillation ratio).

The product remaining after the topping and tailing operations was found to contain 93 %m 8-methyl-7-nonenal 935 g of said product was submitted to a further purification step by distillation in a rotavapor at 127 °C and 10 mbar, which resulted in 728 g of 98.5 %m pure 8-methyl-7-nonenal.

Gas liquid chromatography coupled with mass spectrometry indicated that the product of the hydroformylation reaction contained two compounds both having a molar mass 154, which value corresponds with the molecular weight of 8-methyl-7-nonenal and its isomer 2,7-dimethyl-6-octenal. The compound having the shorter retention time being present in a much lower concentration than the compound with the longer retention time. The latter compound which comprised 98.5 %m of the final purification step resulted in the following 'H-NMR characterization: δ 9.8(s,1H), 5.1(m.1H), 2.4(t.2H), 2.0(2H) and 1.2-1.9(12H).

The outcome of the 'H-NMR spectrum together with hereinbefore described method of preparation, confirms the fact that the compound which was predominantly present in the hydroformylation product is 8-methyl-7-nonenal.

Based on previous and analogous experience with the hydroformylation of mono-α-olefins, which resulted in the formation of a mixture of two isomeric aldehydes it must be concluded that the second compound having a molar mass of 154 is an isomer of 8-methyl-7-nonenal i.e. 2,7-dimethyl-6-octenal.

It is surprising that the process affords 8-methyl-7-nonenal and 2,7-dimethyl-6-octenal with such a high selectivity, and particularly surprising that 8-methyl-7-nonenal is obtained with such a very high selectivity.

## Claims

1. A process for the preparation of an aldehyde of the general formula I, 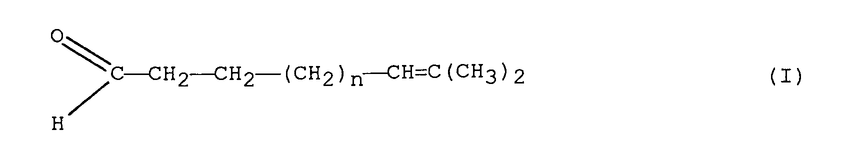 wherein n is an integer of from 2 to 8, which comprises reacting a compound of the general formula II,
CH₂=CH―(CH₂)ₙ―CH=C(CH₃)₂ (II)
wherein n is as defined hereinbefore, with carbon monoxide and hydrogen in the presence of a homogeneous catalyst system comprising a soluble rhodium compound and a phosphor (III) compound in the range of from 50 to 10000 moles per gram atom of rhodium, and in which the temperature is in the range of from 40 to 110°C and the pressure is in the range of from 1.0 to 20 bar.

2. A process as claimed in claim 1, in which n is an integer from 3 to 6.

3. A process as claimed in claim 1 or 2, in which the reaction temperature is from 65 to 80°C and the pressure is in the range of from 1.0 to 3.5 bar.

4. A process as claimed in any of claims 1 to 3, in which the amount of phosphor(III) compound used per gram atom of rhodium is in the range from 500 to 2000 moles, and in which the soluble rhodium compound is a compound of rhodium with one or more of a hydrogen atom, carbon monoxide, a phosphor(III) compound, an olefin and a ketone.

5. A process as claimed in any one of claims 1 to 4, in which the phosphor(III) compound is a phosphine, a phosphite or a phosphor(III) compound in which the phosphor is attached to aryl and/or (cyclo)alkyl groups as well as to aryloxy and/or (cyclo)alkoxy groups, and is preferably a phosphine of the general formula
P(R³)₂R⁴ (III)
in which each R³ independently represents a phenyl or naphthyl group optionally substituted by substituents selected from an alkyl, alkoxy, carboxy, trihalomethyl, cyano, dialkylamino, alkanoyl, alkanoyloxy and an alkylsulphonyl group, and a halogen atom; and R⁴ represents a group R³ or an alkyl or cycloalkyl group; any alkyl group having up to 20 carbon atoms and any cycloalkyl group having up to 12 carbon atoms, more preferably triphenylphosphine.

6. A process as claimed in any one of claims 1 to 5, in which the reaction is performed in the presence of a solvent selected from an amide, a nitrile, an ether, an ester and an aromatic hydrocarbon.

## Patentansprüche

1. Verfahren zur Herstellung von Aldehyden der allgemeinen Formel I, 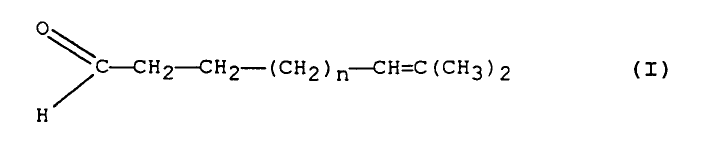 worin n eine ganze Zahl von 2 bis 8 bedeutet, wobei man eine Verbindung der allgemeinen Formel II,
CH₂=CH-(CH₂)ₙ-CH=C(CH₃)₂ (II)
worin n die obenangegebene Bedeutung besitzt, mit Kohlenmonoxid und Wasserstoff in Gegenwart eines homogenen Katalysatorsystems aus einer löslichen Rhodiumverbindung und einer Phosphor(III)-Verbindung in einer Menge von 50 bis 10 000 Mol pro g-Atom Rhodium umsetzt, und wobei die Temperatur im Bereich von 40 bis 110°C und der Druck im Bereich von 1,0 bis 20 bar liegen.

2. Verfahren nach Anspruch 1, wobei n eine ganze Zahl von 3 bis 6 bedeutet.

3. Verfahren nach Anspruch 1 oder 2, wobei die Reaktionstemperatur 65 bis 80°C beträgt und der Druck im Bereich von 1,0 bis 3,5 bar liegt.

4. Verfahren nach einem der Ansprüche 1 bis 3, wobei die pro g-Atom Rhodium eingesetzte Menge an Phosphor(III)-Verbindung im Bereich von 500 bis 2000 Mol liegt und wobei die lösliche Rhodiumverbindung eine Verbindung von Rhodium mit einem oder mehreren aus Wasserstoffatom, Kohlenmonoxid, einer Phosphor(III)-Verbindung, Olefin und Keton ist.

5. Verfahren nach einem der Ansprüche 1 bis 4, wobei die Phosphor(III)-Verbindung ein Phosphin, ein Phosphit oder eine Phosphor(III)-Verbindung ist, bei der der Phosphor an Aryl- und/oder (Cyclo)alkyl- sowie Aryloxy- und/oder (Cyclo)alkoxygruppen gebunden ist und vorzugsweise ein Phosphin der allgemeinen Formel
P(R³)₂R⁴ (III)
ist, worin R³ jeweils unabhängig eine Phenyl- oder Naphthylgruppe, die gegebenenfalls mit Substituenten, ausgewählt aus Alkyl-, Alkoxy-, Carboxyl-, Trihalogenmethyl-, Cyan-, Dialkylamino-, Alkanoyl-, Alkanoyloxy- und Alkylsulfonylgruppen, oder einem Halogenatom substituiert ist, und R⁴ eine Gruppe R³ oder eine Alkyl- oder Cycloalkylgruppe, eine beliebige Alkylgruppe mit bis zu 20 Kohlenstoffatomen und eine beliebige Cycloalkylgruppe mit bis zu 12 Kohlenstoffatomen bedeuten, und besonders bevorzugt Triphenylphosphin ist.

6. Verfahren nach einem der Ansprüche 1 bis 5, wobei die Reaktion in Gegenwart eines Lösungsmittels, ausgewählt aus einem Amid, einem Nitril, einem Ether, einem Ester und einem aromatischen Kohlenwasserstoff, durchgeführt wird.

## Revendications

1. Un procédé pour la préparation d'un aldéhyde de la formule générale I, 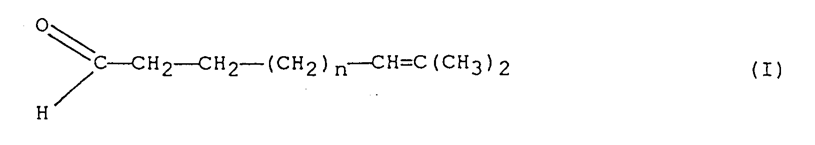 dans laquelle n est un nombre entier de 2 à 8, qui comprend la réaction d'un composé de la formule générale II,
CH₂=CH-(CH₂)ₙ-CH=C(CH₃)₂ (II)
dans laquelle n est tel que défini ci-dessus, avec le monoxyde de carbone et l'hydrogène en présence d'un système catalytique homogène comprenant un composé soluble du rhodium et un composé du phosphore (III) dans une quantité de 50 à 10 000 moles par atome-gramme de rhodium et où la température est comprise entre 40 et 110°C et la pression est comprise entre 1,0 et 20 bars.

2. Un procédé selon la revendication 1, dans lequel n est un nombre entier de 3 à 6.

3. Un procédé selon la revendication 1 ou 2, dans lequel la température de réaction est comprise entre 65 et 80°C et la pression est comprise entre 1,0 et 3,5 bars.

4. Un procédé selon l'une quelconque des revendications 1 à 3, dans lequel la quantité de composé du phosphore (III) utilisée par atome-gramme de rhodium est comprise entre 500 et 2000 moles, et dans lequel le composé soluble du rhodium est un composé du rhodium avec une ou plusieurs des portions suivantes : un atome d'hydrogène, le monoxyde de carbone, un composé du phosphore (III), une oléfine et une cétone.

5. Un procédé selon l'une quelconque des revendications 1 à 4, dans lequel le composé du phosphore (III) est une phosphine, un phosphite ou un composé du phosphore (III) dans lequel le phosphore est attaché à des groupes aryle et/ou (cyclo)alcoyle ainsi qu'à des groupes aryloxy et/ou (cyclo)alcoxy, et est de préférence une phosphine de la formule générale
P(R³)₂R⁴ (III)
dans laquelle chaque R³ indépendamment représente un groupe phényle ou naphtyle éventuellement substitué par des substituants choisis parmi les groupes alcoyle, alcoxy, carboxy, trihalométhyle, cyano, dialcoylamino, alcanoyle, alcanoyloxy et alcoylsulfonyle et les atomes d'halogènes ; et R⁴ représente un groupe R³ ou un groupe alcoyle ou cycloalcoyle ; n'importe quel groupe alcoyle ayant jusqu'à 20 atomes de carbone, de préférence jusqu'à 12 atomes de carbone, et n'importe quel groupe cycloalcoyle ayant jusqu'à 12 atomes de carbone, de préférence jusqu'à 6 atomes de carbone, d'une façon particulièrement préférable la triphénylphosphine.

6. Un procédé selon l'une quelconque des revendications 1 à 5, dans lequel la réaction est conduite en présence d'un solvant choisi parmi un amide, un nitrile, un éther, un ester et un hydrocarbure aromatique.
